# EUROPEAN PATENT APPLICATION

(11) **EP 4 089 104 A1**
(43) Date of publication of application: **16.11.2022**
(21) Application number: 21738947.7
(22) Date of filing: 08.01.2021
(51) Int. Cl.: C07K 14/01, C12N 15/33, C12Q 1/686

(54) **METHOD FOR AMPLIFYING NUCLEIC ACID**

(30) Priority: 10.01.2020 JP 2020002506
(71) Applicant: Kao Corporation, Chuo-ku Tokyo 103-8210 (JP)
(72) Inventor: UEHARA, Yuya, Haga-gun, Tochigi 321-3497 (JP); INOUE, Takayoshi, Haga-gun, Tochigi 321-3497 (JP)
(74) Representative: Vossius & Partner Patentanwälte Rechtsanwälte mbB
(86) International application number: PCT/JP2021/000457
(87) International publication number: WO 2021/141108

(57) **Abstract**

Provided is a method for amplifying a nucleic acid from RNA with high sensitivity and a high yield. The method for amplifying a nucleic acid comprises: synthesizing cDNA from sample RNA through reverse transcription reaction; and amplifying the cDNA by multiplex PCR, wherein the reverse transcription and the multiplex PCR are performed in the presence of a single-stranded nucleic acid-binding protein, and the single-stranded nucleic acid-binding protein is T4GP32 or a mutant thereof.

## Description

### Field of the Invention

The present invention relates to a method for amplifying a nucleic acid.

### Background of the Invention

Multiplex PCR is a method of simultaneously amplifying a plurality of regions from one DNA sample through PCR reaction by using a plurality of primer pairs at the same time. Multiplex RT-PCR, a combination of reverse transcription (RT) reaction from RNA to cDNA and multiplex PCR, is used for gene expression analysis, genotyping, and so on.

RNA is generally extracted from tissue samples. Meanwhile, a method of collecting RNA from skin surface lipid (SSL) has been recently disclosed as a simpler, noninvasive RNA collection method (Patent Literature 1). RNA contained in SSL is useful as an RNA sample for gene expression analysis or the like. However, the amount of SSL which can be collected from one subject is not large, and the amount of RNA contained therein is not large, either; hence, the amount of RNA which can be prepared from SSL of one subject is minute.

Non Patent Literature 1 discloses that BSA and T4 gene 32 protein (T4GP32) improve the reaction of PCR in the presence of an inhibitor. Non Patent Literature 2 discloses that DMSO and betaine provided enhanced specificity and yields in PCR. Non Patent Literatures 3 and 4 disclose that the yields of RT-PCR products significantly increased by adding T4GP32 to the RT system in advance in RT-PCR, but that addition of T4GP32 to the PCR system after RT did not provide any distinctly increased yield. Patent Literature 2 discloses that in reverse transcription with random displacement amplification (RT-RamDA method), in which cDNA is amplified with use of RNA as a template, the yield of cDNA increases by binding T4GP32 to single-stranded cDNA peeled from template RNA to protect the cDNA from degradation by nuclease.
(Patent Literature 1) WO 2018/008319
(Patent Literature 2) WO 2016/052619

(Non Patent Literature 1) Appl Environ Microbiol, 1996, 62 (3) :1102-1106
(Non Patent Literature 2) PLOS ONE, 2010, 5(6):e11024
(Non Patent Literature 3) Appl Environ Microbiol, 1998, 64 (2) :669-677
(Non Patent Literature 4) BioTechniques, 2001, 31(1):81-86

### Summary of the Invention

The present invention provides a method for amplifying a nucleic acid, the method comprising:
synthesizing cDNA from sample RNA through reverse transcription; and
amplifying the cDNA by multiplex PCR, wherein the reverse transcription and the multiplex PCR are performed in the presence of a single-stranded nucleic acid-binding protein, and the single-stranded nucleic acid-binding protein is T4GP32 or a variant thereof.

Further, the present invention provides a yield improver for multiplex RT-PCR, the improver comprising a single-stranded nucleic acid-binding protein as an active ingredient, wherein the single-stranded nucleic acid-binding protein is T4GP32 or a variant thereof, and added to both a reaction solution for reverse transcription and a reaction solution for multiplex PCR.

### Detailed Description of the Invention

All the contents of Patent Literatures, Non Patent Literatures, and other publications cited herein are hereby incorporated by reference in their entirety.

The term "identity of at least 90%" for an amino acid sequence or a nucleotide sequence herein refers to an identity of 90% or higher, preferably of 95% or higher, more preferably of 97% or higher, further more preferably of 98% or higher, further more preferably of 99% or higher.

The identity of nucleotide sequences and amino acid sequences is herein calculated with a Lipman-Pearson method (Science, 1985, 227:1435-41). Specifically, the calculation is performed through analysis with a homology analysis (Search homology) program of the genetic information processing software Genetyx-Win (Ver.5.1.1; Software Development Co., Ltd.) with setting "Unit size to compare (ktup)" to 2.

Achievement of enhanced sensitivity and yield in multiplex RT-PCR is desired. The present invention relates to a method for amplifying a nucleic acid from RNA with high sensitivity and a high yield.

The present inventors found that a high yield can be achieved even from a minute amount of an RNA sample by performing both reactions of RT and PCR in multiplex RT-PCR in the presence of T4GP32.

The present invention provides enhanced sensitivity and yields of multiplex RT-PCR. The method of the present invention enables efficient amplification and analysis of minute amounts of RNA samples such as SSL-derived RNA. The present invention enhances the sensitivity, precision, and efficiency of analysis using RNA samples (e.g., gene analysis, diagnosis).

The present invention provides a method for amplifying a nucleic acid from RNA. The method includes: synthesizing cDNA from sample RNA through reverse transcription; and amplifying the cDNA by multiplex PCR.

The sample RNA to be used in the method of the present invention may be any type of RNA, and examples thereof include RNAs derived from animals, plants, microorganisms, and viruses. The sample RNA may contain mRNA, tRNA, rRNA, small RNA (e.g., microRNA (miRNA), small interfering RNA (siRNA), Piwi-interacting RNA (piRNA)), long intergenic non-coding (linc) RNA, and other types of RNA. One or more of the RNAs listed above may be contained in the sample RNA.

The sample RNA can be extracted, for example, from an animal, a plant, a microorganism, or a virus with a common approach. For the extraction of RNA, for example, the phenol/chloroform method or a modified method thereof, the AGPC (acid guanidinium thiocyanate-phenolchloroform extraction) method or a modified method thereof, a method using special magnetic particles coated with silica, a method using solid-phase reversible immobilization magnetic particles, and commercially available RNA purification reagents (e.g., TRIzol (R) Reagent, RNeasy (R), QIAzol (R), ISOGEN) can be used.

In a preferred embodiment, the sample RNA is RNA derived from skin surface lipid. The term "skin surface lipid (SSL)" herein refers to a liposoluble fraction present on the surface of the skin, and is also called sebum. In general, SSL primarily contains secretions from exocrine glands such as sebaceous glands present in the skin, and exists on the skin surface in the form of a thin layer covering the surface of the skin. SSL contains RNA derived from skin cells of a subject, and use of such SSL allows analysis of the living body (Patent Literature 1).

The subject from which the SSL-derived RNA is to be collected can be an organism having SSL on the skin. Examples of the subject include mammals including humans and non-human mammals, and the subject is preferably a human. For example, the subject may be a human or non-human mammal that needs or wants analysis of his/her or its own nucleic acid. Alternatively, the subject may be a human or non-human mammal that needs or wants gene expression analysis for the skin or analysis of the condition of the skin or a site other than skin with use of a nucleic acid.

Examples of the site of the skin from which SSL is to be collected in a subject include, but are not limited to: skin at any site of the body such as the head, the face, the neck, the trunk, and any of the limbs; skin with a disease such as atopic dermatitis, acne, dryness, inflammation (redness), and tumor; and skin with a wound. Unless otherwise specified, the term "skin" is herein a collective term for regions in the body surface, including the epidermis, the dermis, hair follicles, and tissues including sweat glands, sebaceous glands, and other glands.

SSL from a subject contains RNA expressed in skin cells of the subject, preferably contains RNA expressed in any of the epidermis, sebaceous glands, hair follicles, sweat glands, and dermis of the subject, and more preferably contains RNA expressed in any of the epidermis, sebaceous glands, hair follicles, and sweat glands of the subject (see Patent Literature 1). Accordingly, the SSL-derived RNA to be prepared with the method of the present invention is preferably RNA derived from at least one site selected from the group consisting of the epidermis, sebaceous glands, hair follicles, sweat glands, and dermis of a subject, and more preferably RNA derived from at least one site selected from the group consisting of the epidermis, sebaceous glands, hair follicles, and sweat glands.

To collect SSL from the skin of a subject, any approach for collection or removal of SSL from skin can be employed. Preferably, an SSL-absorbing material, an SSL-adhering material, or an instrument to scrape off SSL from the skin, each described later, can be used. The SSL-absorbing material or SSL-adhering material is not limited as long as the material has affinity with SSL, and examples thereof include polypropylene and pulp. More specific examples of procedures to collect SSL from skin include a method of allowing SSL to be absorbed into a sheet-like material such as an oil-blotting paper and an oil-blotting film, a method of allowing SSL to adhere to a glass sheet, tape, or the like, and a method of collecting SSL by scraping off SSL from the skin with a spatula, a scraper, or other means. For enhanced adsorption of SSL, an SSL-absorbing material impregnated in advance with a highly liposoluble solvent may be used. On the other hand, it is not preferable for SSL-absorbing materials to contain highly water-soluble solvent or moisture because the adhesion of SSL is inhibited. It is preferable to use an SSL-absorbing material in a dry state.

The RNA-containing SSL collected from a subject may be stored until used for RNA preparation described below. The SSL can be stored with the SSL absorbed in or adhering to the SSL-absorbing material or SSL-adhering material. The SSL may be stored under conventional, common conditions for storage of RNA (e.g., at -80°C); however, the SSL can be stored under milder conditions (PCT/JP2019/043040). The temperature condition for storage of the RNA-containing SSL can be, for example, 0°C or lower, preferably -10°C or lower, and more preferably -20°C or lower. The duration of the storage is preferably 12 months or shorter, more preferably 6 months or shorter, and further more preferably 3 months or shorter, though the duration is not limited thereto.

Any of the above-mentioned common approaches for RNA extraction, such as the phenol/chloroform method, the AGPC method, and commercially available reagents, columns, and magnetic particles for RNA purification, can be used for extraction of RNA from SSL.

In the method of the present invention, cDNA is synthesized from the sample RNA through reverse transcription (RT), and the cDNA is then amplified by multiplex PCR (MPCR). The RT reaction and MPCR are both performed in the presence of a single-stranded nucleic acid-binding protein.

The reverse transcription for the sample RNA can be performed by a common procedure, except that a single-stranded nucleic acid-binding protein is added to the reaction system. For example, cDNA can be synthesized by incubating a reaction solution containing the sample RNA, primers, reverse transcriptase, and a substrate to allow the reverse transcriptase to act on the sample RNA. For the primers, primers targeting specific RNA to be analyzed may be used, and it is preferable to use of oligo-dT primers, random primers, or a mixture of them for more comprehensive storage and analysis of nucleic acid. For the reverse transcriptase, a common reverse transcriptase can be used, and it is preferable to use a reverse transcriptase with high accuracy and efficiency in reverse transcription (e.g., M-MLV Reverse Transcriptase and a variant thereof, or commercially available enzymes for reverse transcription reaction described below). The substrate is a substrate for the reverse transcriptase, and deoxyribonucleotide such as dATP, dCTP, dGTP, and dTTP, and a mixture of them can be preferably used. The deoxyribonucleotide may be modified. Examples of enzymes and other reagents for RT in the present invention include commercially available kits of enzymes for reverse transcription reaction and reagents for reverse transcription, such as PrimeScript (R) Reverse Transcriptase series (Takara Bio Inc.) and SuperScript (R) Reverse Transcriptase series (Life Technologies Japan Ltd.), and SuperScript (R) III Reverse Transcriptase and a SuperScript (R) VILO cDNA Synthesis kit (both from Life Technologies Japan Ltd.) can be preferably used. The temperature and time of the RT reaction can be appropriately set, for example, depending on the type of an enzyme or reagent to be used, sample RNA, and cDNA to be synthesized. For example, a reaction solution is prepared using a commercially available reagent for reverse transcription reaction in accordance with the manual, and a single-stranded nucleic acid-binding protein can be added to the reaction solution. The conditions for RT can also be set in accordance with the manual of the reagent.

The cDNA synthesized through the RT is amplified by MPCR. The MPCR can be performed by a common procedure, except that a single-stranded nucleic acid-binding protein is added to the reaction system. For example, a reaction solution containing cDNA, a plurality of primer pairs, DNA polymerase, and a substrate can be subjected to PCR. The primer pairs may be appropriately designed depending on the target DNA sequence. For the DNA polymerase, a common DNA polymerase can be used, and it is preferable to use a DNA polymerase with high accuracy and efficiency in amplification (e.g., a commercially available enzyme for multiplex PCR described later). The substrate is a substrate for the DNA polymerase, and deoxyribonucleotides such as dATP, dCTP, dGTP, and dTTP, and a mixture of them can be preferably used. The deoxyribonucleotides may be modified. Examples of applicable enzymes and other reagents for MPCR in the present invention include commercially available enzymes and reagents for multiplex PCR such as an Ion AmpliSeq Transcriptome Human Gene Expression Kit (Life Technologies Japan Ltd.). The temperature and time of the reaction can be appropriately set, for example, depending on the type of an enzyme or reagent to be used and the template cDNA. For example, a reaction solution is prepared using a commercially available reagent for multiplex PCR in accordance with the manual, and a single-stranded nucleic acid-binding protein can be added to the reaction solution. The conditions for PCR can also be set in accordance with the manual of the reagent.

In the method of the present invention, the RT reaction and MPCR are both performed in the presence of a single-stranded nucleic acid-binding protein. Examples of the single-stranded nucleic acid-binding protein include T4GP32 (T4 gene 32 protein) and variants thereof. T4GP32 is a protein typically consisting of the amino acid sequence of SEQ ID NO: 1, and binds to single-stranded DNA. The variants of T4GP32 may be proteins obtained by subjecting any amino acid in the amino acid sequence of T4GP32 to modification or mutation. Examples of the variants of T4GP32 include a protein consisting of an amino acid sequence having an identity of at least 90% to the amino acid sequence of SEQ ID NO: 1, and being capable of binding to a single-stranded nucleic acid, preferably to single-stranded DNA. T4GP32 can be purchased (from NEW ENGLAND BioLabs Inc., Sigma-Aldrich Co. LLC, etc.). The initial concentration of the single-stranded nucleic acid-binding protein in the reaction solution for the RT and the reaction solution for the MPCR may be preferably 1 µg/mL or more, more preferably 5 µg/mL or more, further more preferably 7 µg/mL or more, and preferably 100 µg/mL or less, more preferably 20 µg/mL or less, further more preferably 15 µg/mL or less, or the initial concentration may be preferably from 1 to 100 µg/mL, more preferably from 5 to 20 µg/mL, further more preferably from 7 to 15 µg/mL.

In the method of the present invention, it is preferable that the RT and MPCR be performed in separate reaction systems (two steps), but they may be performed in one reaction system (one step) for simplicity. In the case of one-step reaction, the single-stranded nucleic acid-binding protein used in the RT can be directly used in the MPCR.

The products obtained through the MPCR are preferably purified by size separation of the reaction products. Size separation allows target PCR products to be separated from primers and other impurities contained in the MPCR reaction solution. Size separation of DNA can be performed, for example, with a size separation column, size separation chips, or magnetic beads applicable to size separation. Preferred examples of magnetic beads applicable to size separation include solid-phase reversible immobilization (SPRI) magnetic beads, such as Ampure XP. When Ampure XP and DNA solutions are mixed together, the DNA is adsorbed on carboxyl groups coating the surfaces of the magnetic beads, and the DNA is purified by collecting only the magnetic beads with a magnet. As the mixing ratio between the Ampure XP solution and the DNA solution is changed, the molecular size of the DNA to be adsorbed on the magnetic beads varies. Use of this principle allows DNA with specific molecular size as a capture target to be collected on the magnetic beads and to be purified from DNA with other molecular size and impurities.

The purified PCR products may be subjected to an additional treatment necessary for the subsequent analysis. For sequencing or fragment analysis of DNA, for example, the purified PCR products are prepared into a proper buffer solution, the PCR primer regions included in the PCR-amplified DNA may be cut out, and an adapter sequence may be further added to the amplified DNA. For example, libraries for various analyses can be prepared by preparing the purified PCR products into a buffer solution, subjecting the amplified DNA to removal of the PCR primer sequences and adapter ligation, and amplifying the resulting reaction products, as necessary. These operations can be performed, for example, by using 5x VILO RT Reaction Mix attached to a SuperScript (R) VILO cDNA Synthesis kit (Life Technologies Japan Ltd.), 5× Ion AmpliSeq HiFi Mix attached to an Ion AmpliSeq Transcriptome Human Gene Expression Kit (Life Technologies Japan Ltd.), and an Ion AmpliSeq Transcriptome Human Gene Expression Core Panel in accordance with the protocols attached to the kits.

The method for amplifying a nucleic acid according to the present invention can be applied to various analyses or diagnoses using RNA. The method of the present invention can be preferably used for any type of analyses to which multiplex PCR can be applied, for example, comprehensive analysis of genes or expression analysis targeting a specific gene, such as gene expression analysis, transcriptome analysis, functional analysis of a subject, diagnosis of a disease, and effect evaluation of a drug administered to a subject.

The following substances, production methods, uses, methods, and others will further be disclosed herein as exemplary embodiments of the present invention. However, the present invention is not limited to these embodiments.
[1] A method for amplifying a nucleic acid, the method comprising:
   synthesizing cDNA from sample RNA through reverse transcription; and
   amplifying the cDNA by multiplex PCR, wherein the reverse transcription and the multiplex PCR are performed in the presence of a single-stranded nucleic acid-binding protein, and the single-stranded nucleic acid-binding protein is T4GP32 or a variant thereof.
[2] The method according to [1], wherein, preferably, the T4GP32 or the variant thereof is a protein consisting of the amino acid sequence of SEQ ID NO: 1 or an amino acid sequence having an identity of at least 90% thereto, and being capable of binding to a single-stranded nucleic acid.
[3] The method according to [1] or [2], wherein the reverse transcription is
   preferably performed in a reaction solution comprising oligo-dT primers, random primers, or a mixture thereof, and
   more preferably performed in a reaction solution comprising random primers.
[4] The method according to any one of [1] to [3], wherein, preferably, the sample RNA is RNA derived from skin surface lipid.
[5] The method according to any one of [1] to [4], wherein an initial concentration of the single-stranded nucleic acid-binding protein in a reaction solution for the reverse transcription and a reaction solution the multiplex PCR is preferably 1 µg/mL or more, more preferably 5 µg/mL or more, further more preferably 7 µg/mL or more, and preferably 100 µg/mL or less, more preferably 20 µg/mL or less, further more preferably 15 µg/mL or less, or the initial concentration is preferably from 1 to 100 µg/mL, more preferably from 5 to 20 µg/mL, further more preferably from 7 to 15 µg/mL.
[6] A yield improver for multiplex RT-PCR, the improver comprising a single-stranded nucleic acid-binding protein as an active ingredient, wherein the single-stranded nucleic acid-binding protein is T4GP32 or a variant thereof, and added to both a reaction solution for reverse transcription and a reaction solution for multiplex PCR.
[7] The improver according to [6], wherein, preferably, the T4GP32 or the variant thereof is a protein consisting of the amino acid sequence of SEQ ID NO: 1 or an amino acid sequence having an identity of at least 90% thereto, and being capable of binding to a single-stranded nucleic acid.
[8] The improver according to [6] or [7], wherein the reverse transcription is
   preferably performed in a reaction solution comprising oligo-dT primers, random primers, or a mixture thereof, and
   more preferably performed in a reaction solution comprising random primers.
[9] The improver according to any one of [6] to [8], wherein, preferably, the improver improves a yield in multiplex RT-PCR using RNA derived from skin surface lipid.
[10] The improver according to any one of [6] to [9], wherein an initial concentration of the single-stranded nucleic acid-binding protein in the reaction solution for the reverse transcription and the reaction solution for the multiplex PCR is preferably 1 µg/mL or more, more preferably 5 µg/mL or more, further more preferably 7 µg/mL or more, and preferably 100 µg/mL or less, more preferably 20 µg/mL or less, further more preferably 15 µg/mL or less, or the initial concentration is preferably from 1 to 100 µg/mL, more preferably from 5 to 20 µg/mL, further more preferably from 7 to 15 µg/mL.
[11] The improver according to any one of [6] to [10], wherein, preferably, the improver improves a sensitivity and yield in multiplex RT-PCR.
[12] Use of a single-stranded nucleic acid-binding protein for improving a yield in multiplex RT-PCR, wherein the single-stranded nucleic acid-binding protein is T4GP32 or a variant thereof, and added to both a reaction solution for reverse transcription and a reaction solution for multiplex PCR.
[13] The use according to [12], wherein, preferably, the T4GP32 or the variant thereof is a protein consisting of the amino acid sequence of SEQ ID NO: 1 or an amino acid sequence having an identity of at least 90% to thereto, and being capable of binding to a single-stranded nucleic acid.
[14] The use according to [12] or [13], wherein the reverse transcription is
   preferably performed in a reaction solution comprising oligo-dT primers, random primers, or a mixture thereof, and
   more preferably performed in a reaction solution comprising random primers.
[15] The use according to any one of [12] to [14], wherein, preferably, the single-stranded nucleic acid-binding protein is used for improving a yield in multiplex RT-PCR using RNA derived from skin surface lipid.
[16] The use according to any one of [12] to [15], wherein an initial concentration of the single-stranded nucleic acid-binding protein in the reaction solution for the reverse transcription and the reaction solution for the multiplex PCR is preferably 1 µg/mL or more, more preferably 5 µg/mL or more, further more preferably 7 µg/mL or more, and preferably 100 µg/mL or less, more preferably 20 µg/mL or less, further more preferably 15 µg/mL or less, or the initial concentration is preferably from 1 to 100 µg/mL, more preferably from 5 to 20 µg/mL, further more preferably from 7 to 15 µg/mL.
[17] The use according to any one of [12] to [16], wherein, preferably, the single-stranded nucleic acid-binding protein is used for improving a sensitivity and yield in multiplex RT-PCR.
[18] Use of a single-stranded nucleic acid-binding protein for production of a yield improver for multiplex RT-PCR, wherein the single-stranded nucleic acid-binding protein is T4GP32 or a variant thereof, and the improver is added to both a reaction solution for reverse transcription and a reaction solution for multiplex PCR.
[19] The use according to [18], wherein, preferably, the T4GP32 or the variant thereof is a protein consisting of the amino acid sequence of SEQ ID NO: 1 or an amino acid sequence having an identity of at least 90% thereto, and being capable of binding to a single-stranded nucleic acid.
[20] The use according to [18] or [19], wherein the reverse transcription is
   preferably performed in a reaction solution comprising oligo-dT primers, random primers, or a mixture thereof, and
   more preferably performed in a reaction solution comprising random primers.
[21] The use according to any one of [18] to [20], wherein, preferably, the single-stranded nucleic acid-binding protein is used for improving a yield in multiplex RT-PCR using RNA derived from skin surface lipid.
[22] The use according to any one of [18] to [21], wherein an initial concentration of the single-stranded nucleic acid-binding protein in the reaction solution for the reverse transcription and the reaction solution for the multiplex PCR is preferably 1 µg/mL or more, more preferably 5 µg/mL or more, further more preferably 7 µg/mL or more, and preferably 100 µg/mL or less, more preferably 20 µg/mL or less, further more preferably 15 µg/mL or less, or the initial concentration is preferably from 1 to 100 µg/mL, more preferably from 5 to 20 µg/mL, further more preferably from 7 to 15 µg/mL.
[23] The use according to any one of [18] to [22], wherein, preferably, the single-stranded nucleic acid-binding protein is used for improving a sensitivity and yield in multiplex RT-PCR.

### Examples

Hereinafter, the present invention will be described in more detail on the basis of Examples, but the present invention is not limited thereto.

### Example 1: Effect of T4GP32 on Multiplex PCR

### 1) Extraction of RNA from SSL

One healthy adult female and one healthy adult male participated as test subjects. Sebum was collected from the overall face of each test subject by using oil-blotting films (5 × 8 cm, made of polypropylene, 3M Japan Limited). The oil-blotting films containing sebum were together transferred into a glass vial, and stored at - 80°C until RNA purification. RNA in SSL contained in the oil-blotting films was purified. The oil-blotting films were cut into pieces of appropriate size, QIAzol (R) Lysis Reagent (QIAGEN) was applied, and an aqueous phase containing RNA was collected from the films. From the collected aqueous phase, RNA was extracted in accordance with the protocol attached to RNeasy (R) (QIAGEN).

### 2) Reverse Transcription and Multiplex PCR

The RNA extracted was subjected to reverse transcription by using a SuperScript (R) VILO cDNA Synthesis kit (Life Technologies Japan Ltd.) at 42°C for 30 minutes to synthesize cDNA from the RNA. The primers used for the reverse transcription reaction were random primers attached to the kit. From the resulting cDNA, a library containing DNAs derived from 20802 genes was prepared by multiplex PCR. The multiplex PCR was carried out by using an Ion AmpliSeq Transcriptome Human Gene Expression Kit (Life Technologies Japan Ltd.) together with a PCR improver under conditions of [99°C, 2 minutes → (99°C, 15 seconds → 60°C, 16 minutes) × 20 cycles → 4°C, Hold]. For the PCR improver, BSA (lyophilized powder, essentially IgG-free, low endotoxin, BioReagent, suitable fwor sell culture) (Sigma-Aldrich Co. LLC, catalog No.: A2058), betaine hydrochloride (FUJIFILM Wako Pure Chemical Corporation), and T4GP32 (NEW ENGLAND BioLabs Inc.) in concentrations shown in Table 1 were used in accordance with the previous reports (Non Patent Literatures 1, 2). The concentration of PCR products of the library prepared was measured by using a TapeStation (Agilent Technologies Japan, Ltd.) and a High Sensitivity D1000 ScreenTape (Agilent Technologies Japan, Ltd.). Relative yields of PCR products to a control (without addition of any PCR improver) were determined.

### 3) Results

Table 1 shows the relative yields of PCR products in the presence of respective PCR improvers. In Table 1, the results for BSA and betaine are those obtained with RNA in SSL derived from the female test subject, and the result for T4GP32 was that obtained with RNA in SSL derived from the male test subject. When T4GP32 was added, relative yield of PCR products to the control increased to 1.41. In contrast, when BSA or betaine was added, PCR products were reduced below the detection limit while PCR products were detected for the control. Although the previous reports (Non Patent Literatures 3, 4) state that addition of T4GP32 to the PCR system after RT did not provide any distinct increase in yield, the addition of T4GP32 is expected to exert significant effect on sensitivity and yields in the reaction in the case of multiplex PCR targeting a plurality of regions as in the present invention.

**[Table 1]**

| **PCR improver** | **Concentration % (w/v)** | **Relative yield of PCR products** |
|---|---|---|
| **Control (without addition)** | **0.0** | **1.00** |
| **BSA** | **0.5** | **undetectable** |
| | **1.0** | **undetectable** |
| **Betaine** | **2.5** | **undetectable** |
| | **5.0** | **undetectable** |
| **T4GP32** | **0.0010** | **1.41** |

### Example 2: Effect of T4GP32 on Reverse Transcription and Multiplex PCR

### 1) Reverse Transcription and Multiplex PCR

One healthy adult male participated as a test subject. Sebum was collected from the overall face of the test subject by using oil-blotting films (5 × 8 cm, made of polypropylene, 3M Japan Limited). RNA was extracted from the oil-blotting films with the same procedure as in Example 1. Reverse transcription was performed under conditions in which T4GP32 was added (0.0010 w/v%) or not added in the same manner as in Example 1. Likewise, multiplex PCR was performed under conditions in which T4GP32 was added (0.0010 w/v%) or not added in the same manner as in Example 1, and the concentration of PCR products was measured. Relative yields of PCR products to a control (without addition of T4GP32 in reverse transcription and PCR) were determined.

### 2) Results

Table 2 shows the results. The relative yields to the control in the case where T4GP32 was added only in reverse transcription and in the case where T4GP32 was added only in multiplex PCR were 1.06 and 1.41, respectively. In contrast to these, the relative yield in the case where T4GP32 was added in both the steps of reverse transcription and multiplex PCR significantly increased to 2.19.

**[Table 2]**

| **T4GP32** | | **Relative yield of PCR products** |
|---|---|---|
| **Reverse transcription** | **Multiplex PCR** | |
| **-** | **-** | **1.00** |
| + | - | **1.06** |
| **-** | + | **1.41** |
| + | + | **2.19** |

## Claims

1. A method for amplifying a nucleic acid, the method comprising:
synthesizing cDNA from sample RNA through reverse transcription; and
amplifying the cDNA by multiplex PCR, wherein the reverse transcription and the multiplex PCR are performed in the presence of a single-stranded nucleic acid-binding protein, and the single-stranded nucleic acid-binding protein is T4GP32 or a variant thereof.

2. The method according to claim 1, wherein the T4GP32 or the variant thereof is a protein consisting of the amino acid sequence of SEQ ID NO: 1 or an amino acid sequence having an identity of at least 90% thereto, and being capable of binding to a single-stranded nucleic acid.

3. The method according to claim 1 or 2, wherein reaction of the reverse transcription is performed in a reaction solution comprising random primers.

4. The method according to any one of claims 1 to 3, wherein the sample RNA is RNA derived from skin surface lipid.

5. The method according to any one of claims 1 to 4, wherein an initial concentration of the single-stranded nucleic acid-binding protein in a reaction solution for the reverse transcription and a reaction solution for the multiplex PCR is from 1 to 100 µg/mL.

6. A yield improver for multiplex RT-PCR, the improver comprising a single-stranded nucleic acid-binding protein as an active ingredient, wherein the single-stranded nucleic acid-binding protein is T4GP32 or a variant thereof, and added to both a reaction solution for reverse transcription and a reaction solution for multiplex PCR.

7. The improver according to claim 6, wherein the T4GP32 or the variant thereof is a protein consisting of the amino acid sequence of SEQ ID NO:1 or an amino acid sequence having an identity of at least 90% thereto, and being capable of binding to a single-stranded nucleic acid.

8. The improver according to claim 6 or 7, wherein the improver improves a sensitivity and yield in multiplex RT-PCR.

9. Use of a single-stranded nucleic acid-binding protein for improving a yield in multiplex RT-PCR, wherein the single-stranded nucleic acid-binding protein is T4GP32 or a variant thereof, and added to both a reaction solution for reverse transcription and a reaction solution for multiplex PCR.

10. The use according to claim 9, wherein the T4GP32 or the variant thereof is a protein consisting of the amino acid sequence of SEQ ID NO: 1 or an amino acid sequence having an identity of at least 90% thereto, and being capable of binding to a single-stranded nucleic acid.

11. The use according to claim 9 or 10, wherein the single-stranded nucleic acid-binding protein is used for improving a sensitivity and yield in multiplex RT-PCR.

12. Use of a single-stranded nucleic acid-binding protein for production of a yield improver for multiplex RT-PCR, wherein the single-stranded nucleic acid-binding protein is T4GP32 or a variant thereof, and the improver is added to both a reaction solution for reverse transcription and a reaction solution for multiplex PCR.

13. The use according to claim 12, wherein the T4GP32 or the variant thereof is a protein consisting of the amino acid sequence of SEQ ID NO: 1 or an amino acid sequence having an identity of at least 90% thereto, and being capable of binding to a single-stranded nucleic acid.

14. The use according to claim 12 or 13, wherein the single-stranded nucleic acid-binding protein is used for improving a sensitivity and yield in multiplex RT-PCR.
